(19) Europäisches Patentamt
European Patent Office
Office européen des brevets

(11) Veröffentlichungsnummer: **0 026 410 A1**

# (12) EUROPÄISCHE PATENTANMELDUNG

(21) Anmeldenummer: 80105636.7

(22) Anmeldetag: 19.09.80

(51) Int. Cl.[3]: **A 61 K 31/66**
//(A61K31/66, 31/395)

(30) Priorität: 28.09.79 GB 7933698
30.07.80 GB 8024890

(43) Veröffentlichungstag der Anmeldung:
08.04.81 Patentblatt 81/14

(84) Benannte Vertragsstaaten:
AT BE CH DE FR GB IT LI LU NL SE

(71) Anmelder: F. HOFFMANN-LA ROCHE & CO.
Aktiengesellschaft

CH-4002 Basel(CH)

(72) Erfinder: Atherton, Frank Ratcliffe
148 Parkway
Welwyn Garden City Herts(GB)

(72) Erfinder: Hall, Michael John
15 Cannonsfield Road
Welwyn Herts(GB)

(72) Erfinder: Hassall, Cedric Herbert
23 The Ryde
Hatfield Herts(GB)

(72) Erfinder: Lambert, Robert Wilson
6 Grange Hill
Welwyn Herts(GB)

(72) Erfinder: Ringrose, Peter Stuart
Geneegasse 10
A-1130 Wien(AT)

(74) Vertreter: Lederer, Franz, Dr. et al,
Patentanwälte Dr. Franz Lederer Reiner F. Meyer
Lucile-Grahn-Strasse 22
D-8000 München 80(DE)

## (54) Wirkstoffkombinationen, deren Herstellung und Verwendung sowie pharmazeutische Präparate.

(57) Wirkstoffkombinationen mit antibiotischen Eigenschaften, dadurch gekennzeichnet, dass sie eine Verbindung der allgemeinen Formel

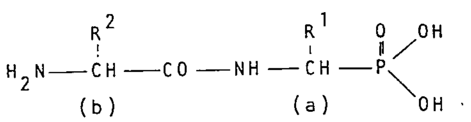

1

worin $R^1$ Wasserstoff, Methyl, Hydroxymethyl, eine Mono-, Di- oder Trihalogenmethylgruppe ist;
$R^2$ einen Niederalkyl-, Hydroxyniederalkyl- oder Guanidinoniederalkylrest, der charakteristisch ist für eine in Proteinen normalerweise nicht vorkommende $\alpha$-Aminosäure, darstellt und
die Konfigurationen an den C-Atomen (a) und (b) R (wenn $R^1 \neq H$) bzw. L sind,
oder ein physiologisch verträgliches Salz einer solchen Verbindung und ein Nocardicin-Antibiotikum enthalten, deren Verwendung und pharmazeutische Präparate sowie deren Herstellung.

EP 0 026 410 A1

0026410

F.Hoffmann-La Roche & Co. Aktiengesellschaft,Basel,Schweiz

Sep. 1980

RAN 4105/55

# Wirkstoffkombinationen, deren Herstellung und Verwendung sowie pharmazeutische Präparate

Die vorliegende Erfindung betrifft Wirkstoffkombinationen mit antibiotischen Eigenschaften, deren Herstellung und Verwendung sowie pharmazeutische Präparate auf deren Basis.

Verbindungen der allgemeinen Formel

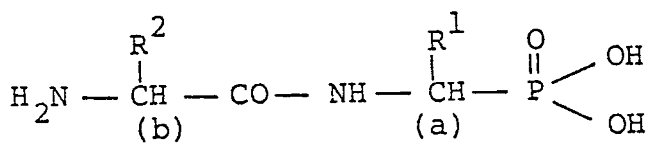

I

worin $R^1$ Wasserstoff, Methyl, Hydroxymethyl, eine Mono-, Di- oder Trihalogenmethylgruppe ist;

$R^2$ einen Niederalkyl-, Hydroxyniederalkyl- oder Guanidinoniederalkylrest, der charakteristisch ist für eine in Proteinen normalerweise nicht vorkommende α-Aminosäure, darstellt und

die Konfigurationen an den C-Atomen (a) und (b) R (wenn $R^1 \neq H$) bzw. L sind,

Mez/27.8.80

und deren physiologisch verträgliche Salze sind bekannt. Diese Verbindungen besitzen antibakterielle Aktivität.

Es wurde nun gefunden, dass Wirkstoffkombinationen, die eine Verbindung der Formel I oder eines ihrer physiologisch verträglichen Salze zusammen mit einem Nocardicin-Antibiotikum enthalten, Kombinationen also, die bisher nicht beschrieben sind, besonders interessant sind im Hinblick auf die Potenzierung eines Nocardicin-Antibiotikums.

In der vorliegenden Anmeldung und den dazugehörigen Ansprüchen bedeutet der Ausdruck "Niederalkyl" gerad- oder verzweigtkettige Alkylreste mit vorzugsweise bis zu 8 Kohlenstoffatomen, wie beispielsweise Ethyl, Propyl, Butyl, tert.Butyl, Pentyl und Hexyl. Beispiele von Hydroxy-Niederalkylgruppen sind Hydroxyethyl, 3-Hydroxypropyl und 4-Hydroxybutyl. Der Ausdruck "Halogen" umfasst Fluor, Chlor, Brom und Jod; Beispiele von Halogenmethylgruppen sind Chlormethyl, Dichlormethyl und Trifluormethyl.

Bevorzugte Verbindungen der Formel I sind solche, in denen $R^1$ Wasserstoff oder Methyl darstellen. Ebenfalls bevorzugt sind Verbindungen der Formel I, in denen $R^2$ Niederalkyl, insbesondere Ethyl, n-Propyl oder n-Butyl, ist. Beispiele der vorstehend definierten Verbindungen der Formel I sind:

(1R)-1-[L-(α-Aminobutyryl)-amino]-ethylphosphonsäure,
(1R)-1-(L-Norleucylamino)-ethylphosphonsäure,
(1R)-1-(L-Norvalylamino)-ethylphosphonsäure,
(L-α-Aminobutyrylamino)-methylphosphonsäure,
(L-Norleucylamino)-methylphosphonsäure,
(L-Norvalylamino)-methylphosphonsäure,
(1R)-1-(L-Homoarginylamino)-ethylphosphonsäure,
(L-Homoarginylamino)-methylphosphonsäure.

Von den vorstehend aufgeführten Verbindungen ist die

Tabelle I

Aktivität von Gemischen von Nocardicin A mit (1R)-1-(L-Norvalylamino)-ethylphosphonsäure gegen Pseudomonas aeruginosa-Stämme

| Stamm No. | Nocardicin A M.I.C. (µg/ml) | Peptid M.I.C. (µg/ml) | Nocardicin A + Peptid | | |
|---|---|---|---|---|---|
| | | | Verh. | M.I.C. (µg/ml) | F.I.C. Index |
| NC1B 8295 | >256 | 64 | 1:1 | 16+16 | <0.31 |
| 511004 | 32 | >256 | 1:8 | 3.6+28.4 | <0.22 |
| 511005 | 4 | 4 | 1:1 | 1+1 | 0.5 |
| 511007 | 16 | >256 | 1:32 | 1.9+62.1 | <0.36 |
| NCTC 2099 | 16 | 256 | 1:8 | 1.8+14.2 | 0.17 |
| NCTC 2100 | 32 | >256 | 1:16 | 3.8+60.2 | <0.35 |
| 511012 | 32 | >256 | 1:8 | 3.6+28.4 | <0.22 |
| 511015 | 16 | 128 | 1:8 | 3.6+28.4 | 0.45 |
| 511016 | 64 | 32 | 1:8 | 1.8+14.2 | 0.47 |
| 511019 | 64 | >256 | 1:8 | 3.6+28.4 | <0.17 |
| 511026 | 181 | >256 | 1:4 | 12.8+51.2 | <0.27 |
| 511027 | 8 | 45 | 1:4 | 3.2+12.8 | 0.68 |
| 511028 | 16 | >256 | 1:16 | 3.8+60.2 | <0.47 |
| 511029 | 256 | >256 | 1:8 | 7.1+56.9 | <0.25 |
| 511030 | 16 | 256 | 1:32 | 1+31 | 0.18 |
| 511032 | 32 | 256 | 1:32 | 1+31 | 0.15 |
| 511033 | 32 | 256 | 1:32 | 1+31 | 0.15 |
| 511036 | 16 | 256 | 1:8 | 3.6+28.4 | 0.33 |
| 511038 | 256 | 256 | 1:2 | 21.3+42.7 | 0.25 |
| 511041 | 32 | 256 | 1:8 | 3.6+28.4 | 0.22 |

Tabelle II

Aktivität von Gemischen von Nocardicin A mit (1R)-1-(L-Norvalylamino)-ethylphosphonsäure in verschiedenen Verhältnissen bei Pseudomonas aeruginosa-Stämmen

| Stamm No. | Nocardicin A + Peptid | | |
|---|---|---|---|
| | Verh. | M.I.C. (µg/ml) | F.I.C. Index |
| NCTC 2099 | 1:0 | 16 | - |
| | 4:1 | 12.8+3.2 | 0.81 |
| | 2:1 | 10.7+5.3 | 0.69 |
| | 1:1 | 8+8 | 0.53 |
| | 1:2 | 5.3+10.7 | 0.37 |
| | 1:4 | 3.2+12.8 | 0.25 |
| | 1:8 | 1.8+14.2 | 0.17 |
| | 1:16 | 1.9+30.1 | 0.24 |
| | 1:32 | 1+31 | 0.18 |
| | 0:1 | 256 | - |
| 511019 | 1:0 | 64 | - |
| | 4:1 | 25.6+6.4 | <0.42 |
| | 2:1 | 21.3+10.7 | <0.37 |
| | 1:1 | 16 +16 | <0.31 |
| | 1:2 | 10.7+21.3 | <0.25 |
| | 1:4 | 6.4+25.6 | <0.2 |
| | 1:8 | 3.6+28.4 | <0.17 |
| | 1:16 | 3.8+60.2 | <0.29 |
| | 1:32 | 1.9+62.1 | <0.27 |
| | 0:1 | >256 | - |
| 511041 | 1:0 | 32 | - |
| | 4:1 | 25.6+6.4 | 0.82 |
| | 2:1 | 21.3+10.7 | 0.71 |
| | 1:1 | 16 +16 | 0.56 |
| | 1:2 | 10.7+21.3 | 0.42 |
| | 1:4 | 6.4+25.6 | 0.3 |
| | 1:8 | 3.6+28.4 | 0.22 |
| | 1:16 | 3.8+60.2 | 0.35 |
| | 1:32 | 1.9+62.1 | 0.30 |
| | 0:1 | 256 | - |

Tabelle III

Aktivität von Gemischen von Nocardicin A mit (1R)-1-(L-Norvalylamino)-ethylphosphonsäure gegen Proteus- und Serratia-Spezies

| Spezies | Stamm No. | Nocardicin A M.I.C.(µg/ml) | Peptide M.I.C. (µg/ml) | Nocardicin A + Peptid | | |
|---|---|---|---|---|---|---|
| | | | | Verh. | M.I.C. (µg/ml) | F.I.C. Index |
| P. mirabilis | 502010 | 8 | 22.6 | 1:4 | 1.6 + 6.4 | 0.48 |
| " " | 502012 | 8 | 22.6 | 1:1 | 2 + 2 | 0.34 |
| " " | 502015 | 4 | 22.6 | 1:16 | 0.5 + 7.5 | 0.46 |
| " " | 502038 | 32 | 22.6 | 4:1 | 12.8 + 1.6 | 0.47 |
| P. morganii | 501041 | 8 | 64 | 1:4 | 3.2 + 12.8 | 0.6 |
| P. rettgeri | 503010 | 8 | 4 | 16:1 | 3.8 + 0.2 | 0.52 |
| P. vulgaris | 505030 | 4 | 5.7 | 1:16 | 0.2 + 3.8 | 0.72 |
| " " | 505031 | 32 | 8 | 2:1 | 5.3 + 2.7 | 0.5 |
| S. marcescens | ATCC 14756 | 8 | 1 | 16:1 | 1.9 + 0.1 | 0.34 |
| " " | 542007 | 11.3 | 1 | 1:1 | 0.5 + 0.5 | 0.54 |
| " " | 542010 | 11.3 | 1 | 1:1 | 0.5 + 0.5 | 0.54 |
| " " | 542012 | 8 | 1 | 2:1 | 0.7 + 0.3 | 0.39 |
| " " | 542017 | 16 | 1 | 16:1 | 3.8 + 0.2 | 0.44 |

Tabelle IV

Aktivität von Gemischen von Nocardicin A mit 1-(L-Norvalylamino)-methylphosphonsäure gegen Pseudomonas aeruginosa, Proteus vulgaris und Serratia marcescens.

| Mikroorganismus | M.I.C. (µg/ml) | | | Nocardicin A / Peptid | F.I.C. Index |
|---|---|---|---|---|---|
| | Nocardicin A | Peptid | Gemisch | | |
| Ps. aeruginosa | 8 | >128 | 16 | 1:16 | <0.24 |
| P. vulgaris | 64 | 45.2 | 8 | 1:2 | 0.16 |
| S. marcescens | 16 | 4 | 4 | 4:1 | 0.4 |

Die in vitro Aktivität der erfindungsgemässen Kombinationen kann auch nach der Mikrotiter-Schachbrett-Methode gezeigt werden. Bei dieser Methode werden Filter-sterilisierte Grundlösungen von Verbindungen I und einem Nocardicin-Antibiotikum hergestellt, die 4,8mal so konzentriert sind wie die zu testenden Lösungen. Aliquote Teile des Kulturmediums (50 µl) wurden in alle 12 (1-12) x 8 (A-H) Löcher der Mikrotiter-Platte gegeben, mit Ausnahme des Loches 12H. Jeweils 50 µl der Peptid-Lösung wurden dann in jedes Loch der zwölften Reihe gegeben und 100 µl in das Loch 12H. Die peptidhaltigen Lösungen wurden dann innerhalb der Reihen A-H dadurch verdünnt, dass man jeweils 50 µl aus der Reihe 12 in die Reihe 11 übertrug, gut vermischte, und wieder jeweils 50 µl in die Reihe 10 übertrug und dieses Prozedere bis zur Reihe 2 wiederholte, so dass die Reihe 1 ausgelassen wurde. Dann wurden je 50 µl einer Lösung eines Nocardicin-Antibiotikums in jedes Loch der Reihe H gegeben und ebenfalls in der vorstehend beschriebenen Weise verdünnt innerhalb der Reihen 1-12, wobei das letzte Loch in jeder Reihe wiederum ausgelassen wurde. Nachdem die Volumina überall durch Zusatz von 50 µl Nährflüssigkeit auf 100 µl gebracht wurden, wurden jeweils 20 µl einer 1/1000 Verdünnung einer über Nacht angesetzten Kultur eines Testorganismus zugesetzt. Durch Schütteln wurde für eine gleichmässige Verteilung gesorgt, woraufhin die Platten mit Klebstreifen verschlossen wurden. Die minimalen Hemmkonzentrationen wurden als diejenigen minimalen Konzentrationen bestimmt, die nach 18-stündiger Inkubation der Kulturen eine sichtbare Trübung verhinderten. Die Ergebnisse mit (1R)-1-(L-Norvalylamino)-ethylphosphonsäure und Nocardicin D sind in der Tabelle V zusammengestellt.

Tabelle V

Aktivität von Gemischen von Nocardicin D mit (1R)-1-(L-Norvalylamino)-ethylphosphonsäure gegen Pseudomonas aeruginosa-Stämme

| Stamm No. | M.I.C. (μg/ml) | | | Nocardicin A / Peptid | F.I.C. Index |
|---|---|---|---|---|---|
| | Nocardicin D | Peptid | Gemisch | | |
| 511019 | >512 | >256 | 192 | 2:1 | <0.25 |
| 511027 | 512 | 64 | 64 | 1:1 | 0.56 |
| 511030 | 128 | 256 | 72 | 1:8 | 0.31 |

Die in vivo Aktivitäten der erfindungsgemässen Kombinationen wurden mittels Pseudomonas-Septikämien an Mäusen getestet, wobei die Verabreichung eine Stunde, 3 Stunden und 5 Stunden nach intraperitonealer Infektion erfolgte. Die Ergebnisse, die mit (1R)-1-(L-Norvalylamino)-ethylphosphonsäure und Nocardicin A erreicht wurden, sind in der folgenden Tabelle VI zusammengefasst.

Tabelle VI

| Mikroorganismus | $CD_{50}$ (mg/kg s.c. 3 x) | | | Gew.-Verhältnis | F.I.C. Index |
|---|---|---|---|---|---|
| | Nocardicin A | Peptid | Gemisch | | |
| Ps. aeruginosa BA | 43 | >200 | 18 + 18 | 1:1 | ≤0.51 |
| " " 143724 | >50 | >200 | 25 + 25 | 1:1 | ≤0.63 |
| " " 143811 | >50 | >200 | 18 + 18 | 1:1 | ≤0.45 |
| " " M 7801 | >50 | 135 | 15 + 15 | 1:1 | ≤0.41 |

Die erfindungsgemässen Wirkstoffkombinationen können in Form pharmazeutischer Präparate verabreicht werden, die ebenfalls Gegenstand der vorliegenden Erfindung sind. Es kommen die üblichen mit den Verbindungen der Formel I bzw. ihren Salzen und den Nocardicin-Antibiotika verträglichen pharmazeutischen Träger für die Herstellung der Präparate in Frage. Trägermaterialien können flüssig oder fest, organischen oder anorganischen Charakters sein und umfassen beispielsweise Wasser, Gelatine, Mannit, mineralische Oele, vegetabile Oele, Gummi arabicum, Propylenglycole oder Polyalkylenglykole.

Die Herstellung der pharmazeutischen Präparate kann in an sich bekannter Weise geschehen, dadurch z.B., dass man die einzelnen Komponenten mit den geeigneten Trägermaterialien vermischt und in eine geeignete galenische Form bringt.

Die pharmazeutischen Präparate können in fester Form (z.B. als Lyophilisate) oder in flüssiger Form (z.B. als Lösungen, Suspensionen oder Emulsionen) vorliegen. Gegebenenfalls sind sie sterilisiert und bzw. oder enthalten weitere Hilfsstoffe wie Konservierungsmittel, Stabilisierungs-, Netz- oder Emulgiermittel, Mittel zur geschmacklichen Verbesserung, Salze zur Veränderung des osmotischen

Druckes oder Puffersubstanzen. Bei Verwendung von Puffern kann der pH-Wert der Präparate innerhalb der üblichen Grenzen variieren.

Der Gehalt an den erfindungsgemässen Wirkstoffkombinationen in pharmazeutischen Präparaten kann innerhalb weiter Grenzen variieren. Die optimale tägliche Dosis hängt ab von den tatsächlich verwendeten Komponenten, dem Applikationsweg, der Art der Infektion, usw. So liegt beispielsweise die tägliche Dosis bei parenteraler Applikation bei etwa 200-2000 mg des Gemisches der aktiven Komponenten. Diese Dosis kann als einmalige Dosis oder in Teildosen verabreicht werden und kann in besonderen Situationen je nach Verschreibung des Arztes aufgrund individueller Erfordernisse erhöht oder erniedrigt werden.

## Beispiel 1

Gemisch zur Herstellung von Injektionslösungen, enthaltend die folgenden Bestandteile:

| | |
|---|---|
| Nocardicin A-Natriumsalz | 200,0 mg |
| (1R)-1-(L-Norvalylamino)-ethylphosphonsäure | 200,0 mg |
| Trinatriumcitrat | 200,0 mg |

Die gemahlenen einzelnen Bestandteile wurden sorgfältig miteinander vermischt und unter sterilen Bedingungen in geeignete Behälter abgefüllt. Zur Herstellung einer Injektionslösung kann das erhaltene Gemisch in 2 ml Wasser für Injektionszwecke gelöst werden.

## Beispiel 2

Lyophilisat zur Herstellung von Injektionslösungen, enthaltend die folgenden Bestandteile:

| | |
|---|---|
| Nocardicin A-Natriumsalz | 200,0 mg |
| (1R)-1-(L-Norvalylamino)-ethylphosphonsäure | 200,0 mg |
| Trinatriumcitrat | 200,0 mg |
| D-Mannit | 200,0 mg |

Eine wässrige Lösung dieser Bestandteile wurde gefriergetrocknet. Zur Herstellung einer Injektionslösung wurde das erhaltene Lyophilisat in 2 ml Wasser für Injektionszwecke gelöst.

Beispiel 3

Es wird eine Injektionslösung hergestellt durch Auflösung von 100 ml Nocardicin A-Natriumsalz in 1 ml einer Lösung, die 100 mg (1R)-1-(L-Norvalylamino)-ethylphosphonsäure pro ml eines geeigneten Puffers enthält. Ein geeigneter Puffer kann folgendermassen zusammengesetzt werden:

| | |
|---|---|
| Natriumchlorid | 8,3 mg |
| Trinatriumcitrat | 50,0 mg |
| Dinatriumcitrat | 50,0 mg |
| Wasser für Injektionszwecke ad | 1,00 ml |

Beispiel 4

Ein pulvriges Gemisch zur Herstellung von Injektionslösungen kann folgende Zusammensetzung aufweisen:

| | |
|---|---|
| Nocardicin A-Natriumsalz | 200,0 mg |
| 1-(L-Norvalylamino)-methyl-phosphonsäure | 200,0 mg |
| Trinatriumcitrat | 200,0 mg |

Dieses Gemisch wird in zu Beispiel 1 analoger Weise hergestellt. Zur Herstellung einer Injektionslösung wird das Gemisch in 2 ml Wasser für Injektionszwecke gelöst.

Beispiel 5

Pulvriges Gemisch für die Herstellung von Injektionslösungen der folgenden Zusammensetzung:

| | |
|---|---|
| Nocardicin D-Natriumsalz | 200,0 mg |
| (1R)-1-(L-Norvalylamino)-ethyl-phosphonsäure | 200,0 mg |
| Trinatriumcitrat | 200,0 mg |

Das Gemisch wird in zu Beispiel 1 analoger Weise hergestellt. Zur Herstellung einer Injektionslösung wird das Gemisch in 2 ml Wasser für Injektionszwecke gelöst.

## Patentansprüche

1. Wirkstoffkombinationen mit antibiotischen Eigenschaften, dadurch gekennzeichnet, dass sie eine Verbindung der allgemeinen Formel

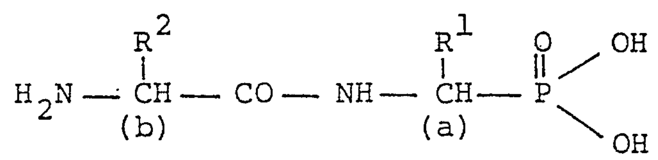

I

worin $R^1$ Wasserstoff, Methyl, Hydroxymethyl, eine Mono-, Di- oder Trihalogenmethylgruppe ist;
$R^2$ einen Niederalkyl-, Hydroxyniederalkyl- oder Guanidinoniederalkylrest, der charakteristisch ist für eine in Proteinen normalerweise nicht vorkommende α-Aminosäure, darstellt und
die Konfigurationen an den C-Atomen (a) und (b) R (wenn $R^1 \neq H$) bzw. L sind,
oder ein physiologisch verträgliches Salz einer solchen Verbindung und ein Nocardicin-Antibiotikum enthalten.

2. Wirkstoffkombinationen gemäss Anspruch 1, dadurch gekennzeichnet, dass das Nocardicin-Antibiotikum Nocardicin A ist.

3. Wirkstoffkombinationen gemäss Anspruch 1 oder 2, dadurch gekennzeichnet, dass in der Verbindung der Formel I $R^1$ Wasserstoff oder Methyl ist.

4. Wirkstoffkombinationen gemäss einem der Ansprüche 1-3, dadurch gekennzeichnet, dass in der Verbindung der Formel I $R^2$ ein Niederalkylrest ist.

5. Wirkstoffkombinationen gemäss Anspruch 4, dadurch gekennzeichnet, dass die Verbindung der Formel I (1R)-1-(L-Norvalylamino)-ethylphosphonsäure ist.

6. Wirkstoffkombinationen gemäss einem der Ansprüche 1-5 zur Therapie und Prophylaxe bakterieller Infektionen.

7. Verfahren zur Herstellung von Wirkstoffkombinationen gemäss einem der Ansprüche 1-5, dadurch gekennzeichnet, dass man eine Verbindung der Formel I oder ein physiologisch verträgliches Salz davon und ein Nocardicin-Antibiotikum miteinander vermischt.

8. Pharmazeutische Präparate auf der Basis einer Wirkstoffkombination gemäss einem der Ansprüche 1-5.

9. Verwendung einer Wirkstoffkombination gemäss einem der Ansprüche 1-5 als antibakterielles Mittel.

***

0026410

Europäisches Patentamt

**EUROPÄISCHER RECHERCHENBERICHT**

Nummer der Anmeldung
EP 80 10 5636

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | US - A - 4 134 972 (F.R. ATHERTON)<br>* Spalte 1 *<br>-- | 1,3 |
| | DE - A - 2 758 867 (FUJISAWA)<br>* Ansprüche *<br>-- | 1,2,6 |
| | EP - A - 0 003 618 (FUJISAWA)<br>* Ansprüche 1,4-7 *<br>---- | 1,2,6 |

KLASSIFIKATION DER ANMELDUNG (Int. Cl.³)

A 61 K 31/66
//(A 61 K 31/66
31/395)

RECHERCHIERTE SACHGEBIETE (Int. Cl.³)

A 61 K 31/66
31/395
C 07 F 9/00
C 07 D 205/08

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 12.12.1980 | CHOULY |